# EUROPÄISCHE PATENTSCHRIFT

(11) **EP 2 488 478 B1**
(45) Veröffentlichungstag und Bekanntmachung des Hinweises auf die Patenterteilung: **25.11.2015**
(21) Anmeldenummer: 10742161.2
(22) Anmeldetag: 13.08.2010
(51) Int. Cl.: C07C 29/17, C07C 31/125

(54) **VERFAHREN ZUR HERSTELLUNG VON DECANOLEN DURCH HYDRIERUNG VON DECENALEN**
METHOD FOR PRODUCING DECANOLS BY MEANS OF HYDROGENATING DECENALS
PROCÉDÉ DE PRODUCTION DE DÉCANOLS PAR HYDROGÉNATION DE DÉCÉNALS

(30) Priorität: 15.10.2009 DE 102009045718
(43) Veröffentlichungstag der Anmeldung: 22.08.2012
(73) Patentinhaber: Evonik Degussa GmbH, 45128 Essen (DE)
(72) Erfinder: KAIZIK, Alfred, 45772 Marl (DE); LUEKEN, Hans-Gerd, 45770 Marl (DE)
(86) Internationale Anmeldenummer: PCT/EP2010/061827
(87) Internationale Veröffentlichungsnummer: WO 2011/045102

(56) Entgegenhaltungen:
- EP-A2- 0 470 344
- DE-A1-102007 041 380
- GB-A- 1 244 442

## Beschreibung

Die vorliegende Erfindung betrifft ein Verfahren zur Herstellung mindestens eines Decanols durch Hydrierung mindestens eines Decenals, bei welchem eine erste Hydrierung in der flüssigen Phase an einem festen ersten Katalysator erfolgt, wobei der erste Katalysator Kupfer und Nickel enthält.

Bekannt ist ein derartiges Verfahren aus DE102007041380A1.

Aus GB 1 244 442 A bekannt ist ein Verfahren zur Hydrierung ungesättigter Aldehyde, bei dem auf eine erste Hydrierung an einem Kupfer und/oder Nickel enthaltenden Katalysator eine zweite Hydrierung an einem Nickel und/oder Palladium enthaltenden Katalysator erfolgt.

Decanole im Sinne dieser Erfindung sind gesättigte Alkohole, die zehn Kohlenstoffatome aufweisen. Verteter der Gruppe der Decanole sind: 2-Propylheptanol, 4-Methyl-2-propyl-hexanol, 5-Methyl-2-propyl-hexanol, 2-isopropyl-4-methyl-hexanol, 2-isopropyl-5-methyl-hexanol, wobei die aufgezählten Decanole jeweils aus mindestens zwei Stereoisomeren bestehen.

Decanole dienen als Vorstufen für die Herstellung von Weichmachern sowie als Zwischenprodukte für die Herstellung von Detergenzien.

Decenale im Sinne dieser Erfindung sind α,β-ungesättigte Aldehyde mit zehn Kohlenstoffatomen. Sie werden insbesondere durch Aldolisierung der gesättigten C₅-Aldehyde (Pentale) gewonnen. Verteter der Gruppe der Decenale sind: 2-Propylheptenal, 4-Methyl-2-propyl-hexenal, 5-Methyl-2-propyl-hexenal, 2-Isopropyl-4-methyl-hexenal, 2-Isopropyl-5-methyl-hexenal.

Es ist bekannt, Carbonylverbindungen, insbesondere Aldehyde, katalytisch mit Wasserstoff zu Alkoholen zu reduzieren. Dabei werden häufig Katalysatoren eingesetzt, die mindestens ein Metall aus den Gruppen 1 b, 2b, 6b, 7b, und/oder 8 des Periodensystems der Elemente enthalten. Die Hydrierung von Aldehyden kann kontinuierlich oder diskontinuierlich mit pulverförmigen oder stückigen Katalysatoren in der Gas- oder Flüssigphase durchgeführt werden.

Für die technische Herstellung von Alkoholen durch Hydrierung von Aldehyden aus dem Oxo-Prozess (Hydroformylierung von Olefinen) oder aus Aldolkondensation von Aldehyden werden, vor allem bei Großprodukten, kontinuierliche Verfahren mit im Festbett angeordneten Katalysatoren in der Gas- oder Flüssigphase angewendet.

Im Vergleich zur Gasphasenhydrierung weist die Flüssigphasenhydrierung die günstigere Energiebilanz und die höhere Raum-Zeit-Ausbeute auf. Mit steigender Molmasse des zu hydrierenden Aldehyds, d. h. mit steigenden Siedepunkten, nimmt der Vorteil der günstigeren Energiebilanz zu. Höhere Aldehyde mit mehr als 7 Kohlenstoffatomen werden demnach vorzugsweise in der Flüssigphase hydriert.

Die Hydrierung in der Flüssigphase hat allerdings den Nachteil, dass aufgrund der hohen Konzentrationen sowohl an Aldehyden als auch an Alkoholen die Bildung von Hochsiedern durch Folge- und Nebenreaktionen begünstigt wird. So können Aldehyde leichter Aldolreaktionen (Addition und/oder Kondensation) eingehen und mit Alkoholen Halb- oder Vollacetale bilden. Die entstandenen Acetale können unter Abspaltung von Wasser oder Alkohol Enolether bilden, die unter den Reaktionsbedingungen zu den gesättigten Ethern hydriert werden. Diese sekundären Nebenprodukte vermindern somit die Ausbeute. Die als Hochsieder bezeichneten Nebenprodukte können bestenfalls teilweise in nachgeschalteten Anlagen in Wertprodukte, wie Ausgangsaldehyde und Zielalkohole, zurückgespalten werden.

Technische Aldehydgemische, die zur Hydrierung eingesetzt werden, enthalten häufig bereits Hochsieder in unterschiedlicher Konzentration.

Die Hydrierung von gesättigten Aldehyden (Hydroformylaten) unter Zusatz von Wasser ist beispielsweise in den Schriften US 5059710, US4401384, GB2142010, US2809220, DE19842370A1 und DE10062448A1 dargelegt.

Diese Verfahren sind für die Hydrierung von Decenalen zu Decanolen nur bedingt geeignet, da durch das vorhandene Wasser zumindest ein Teil der Decenale zu einem oder mehreren C₅-Aldehyd(en) zurückgespalten wird.

Im Vergleich zur Hydrierung von gesättigten Aldehyden ist die Hydrierung von ungesättigten Aldehyden zu gesättigten Alkoholen deutlich komplexer, da in diesem Fall nicht nur die Carbonylgruppe, sondern auch die olefinische Doppelbindung hydriert werden muss.

Ein gattungsbildendes Verfahren zur Hydrierung von gesättigten oder α,β-ungesättigten Aldehyden zu den entsprechenden gesättigten Aldehyden wird in DE102007041380A1 offengelegt. Dabei wird als Hydrierkatalysator ein Trägerkatalysator eingesetzt, der neben den Aktivkomponenten Kupfer und Nickel sowie Chrom als Promotor zusätzlich Bariumoxid enthält.

Bei der Hydrierung von Decenalen zu Decanolen an den in DE102007041380A1 beschriebenen Katalysatoren mit Bariumoxid im Langzeittest zeigte sich, dass nach über 1000 Stunden Betriebszeit im Hydrieraustrag Decenale vorhanden sind.

Decenale im Decanolgemisch können bei dessen Weiterverarbeitung nachteilig sein. Beispielsweise kann bei der Herstellung von Weichmachern aus dem Decanolgemisch durch Reaktion der Decenale ein Produkt mit erhöhter Farbzahl entstehen, sodass zur Senkung der Farbzahl ein erhöhter Aufarbeitungsaufwand notwendig ist.

Aus diesem Grunde ist es wünschenswert, ein Decanolgemisch bereitzustellen, das möglichst frei von Decenalen ist. Da Decenale und Decanole jedoch eine ähnliche Siedelage haben, ist eine physikalische Abscheidung von nicht umgesetzten Decenalen aus dem Decanolgemisch unwirtschaftlich.

Daher liegt der Erfindung die Aufgabe zu Grunde, ein Verfahren der eingangs genannten Gattung anzugeben, gemäß welchem selbst nach langen Betriebszeiten Decenale mit hohen Ausbeuten zu Decanolen zu hydrieren sind. Insbesondere soll der Gehalt an nicht umgesetzen Decenalen im Hydrieraustrag weniger als 1500 ppm, vorzugsweise weniger als 1000 ppm und ganz besonders bevorzugt weniger als 600 ppm betragen.

Gelöst wird diese Aufgabe dadurch, dass die Hydrierung zweistufig erfolgt, nämlich in einer ersten Stufe in an sich bekannter Weise an einem Katalysator enthaltend Kupfer, Nickel und ggf. Chrom und/oder Bariumoxid und darauffolgend in einer zweiten Stufe mit einem anderen Katalysator, welcher frei von Kupfer, Chrom und Nickel sein und Ruthenium enthalten muss.

Gegenstand der Erfindung ist mithin ein Verfahren zur Herstellung mindestens eines Decanols durch Hydrierung mindestens eines Decenals, bei welchem eine erste Hydrierung in der flüssigen Phase an einem festen ersten Katalysator erfolgt, wobei der erste Katalysator Kupfer und Nickel enthält, mit einer auf die erste Hydrierung folgenden zweiten Hydrierung in der flüssigen Phase an einem festen zweiten Katalysator, wobei der zweite Katalysator frei von Kupfer, Chrom und Nickel ist und Ruthenium enthält.

Das erfindungsgemäße Verfahren weist im Vergleich zu einem Verfahren, das nur einen Katalysator verwendet, folgende Vorteile auf: Bei gleichem Gesamtkatalysatorvolumen wird bei vergleichbaren Ausbeuten ein Produkt mit geringerem Gehalt an olefinischen Verbindungen erhalten. Dies hat zur Folge, dass unter Einhaltung eines Grenzwertes an olefinischen Verbindungen im Hydrieraustrag ein geringeres spezifisches Katalysatorvolumen notwendig ist. Wegen der geringeren Katalysatorkosten und geringerer Frequenz des Katalysatorwechsels und damit verbundenen Aufwands ergeben sich geringere spezifische Hydrierkosten. Zudem werden Ausfallzeiten der Produktion durch Katalysatorwechsel verringert.

Einsatzstoffe für das erfindungsgemäße Verfahren sind Decenale (α,β-ungesättigte C₁₀-Aldehyde). Insbesondere werden α,β-ungesättigte C₁₀-Aldehyde eingesetzt, die durch Aldolkondensation von C₅-Aldehyden hergestellt worden sind, wie beispielsweise 2-Propylhept-2-enal aus n-Pentanal und 2-Methyl-2-Isopropylhex-2-enal aus 3-Methylbutanal oder ein Gemisch isomerer Decenale, die durch Kondensation von mindestens zwei verschiedenen C₅-Aldehyden erhalten werden, wie zum Beispiel 4-Methyl-2-Propyl-Hexanal aus Kreuz-Aldolisierung von n-Pentanal mit 2-Methylbutanal. Ganz besonders bevorzugt werden Decenalgemische mit einem Gehalt an 2-Propylhept-2-enal von über 85 Massen-% nach dem erfindungsgemäßen Verfahren hydriert.

Ein Verfahren zur Herstellung solcher Gemische aus C₅-Aldehyden wird beispielsweise in DE102009001594A1 beschrieben.

Im erfindungsgemäßen Verfahren kann für die erste Hydrierstufe ein Trägerkatalysator verwendet, der die hydrieraktiven Metalle Kupfer, Chrom und Nickel sowie Bariumoxid aufweist. Solche Katalysatoren sind in DE102007041380A1 beschrieben. Das Trägermaterial kann Titandioxid, Zirkondioxid, Aluminiumoxid, Siliziumdioxid oder deren Mischoxide sein. Ein bevorzugter Träger ist Aluminiumoxid, vorzugsweise γ-Aluminiumoxid mit BET-Oberflächen zwischen 90 und 200 m²/g. Ein für die erste Hydrierstufe des erfindungsgemäßen Verfahrens einsetzbarer (erster) Katalysator enthält 1 bis 20 Massen-% Kupfer, 0,2 bis 6 Massen-% Chrom, 1 bis 20 Massen-% Nickel, jeweils berechnet als Metall, und 0,1 bis 2 Massen-% Barium, berechnet als Bariumoxid.

In einer bevorzugten Ausführungsform enthält der Trägerkatalysator für die erste Hydrierstufe des erfindungsgemäßen Verfahrens lediglich Kupfer und Nickel als hydrieraktive Metalle, ist also frei von Chrom. Als bevorzugter Träger wird Aluminiumoxid, vorzugsweise γ-Aluminiumoxid mit BET-Oberflächen zwischen 90 und 200 m²/g, eingesetzt. Die genannten Katalysatoren enthalten in der Regel 1 bis 20 Massen-% Kupfer und 1 bis 20 Massen-% Nickel, jeweils berechnet als Metall, insbesondere 6 bis 12 Massen-% Kupfer und 3 bis 8 Massen-% Nickel. Überraschenderweise zeigt sich, dass bei der erfindungsgemäßen, zweistufigen Hydrierung der erste Katalysator sogar frei von Bariumoxid sein kann, ohne dass es zu einer nennenswerten Bildung von Acetalen kommt.

Somit handelt es sich bei dem in dem erfindungsgemäßen Verfahren einzusetzenden Katalysator der ersten Hydrierstufe insbesondere um einen Katalysator aus Kupfer und Nickel, als hydrieraktive Metalle, auf einem Träger, d. h. ohne Zusatz von Chrom und/oder Bariumoxid.

Für die zweite Hydrierstufe des erfindungsgemäßen Verfahrens werden Katalysatoren verwendet, die als hydrieraktives Metall Ruthenium aufweisen.

Als Hydrierkatalysatoren für die zweite Stufe des erfindungsgemäßen Verfahrens können bekannte Ruthenium-Trägerkatalysatoren, die üblicherweise 0,1 bis 10 Massen-% Ruthenium aufweisen, eingesetzt werden. Beispielsweise können Katalysatoren, deren Herstellungen in DE10054347A1 und DE10232868A1 beschrieben sind, eingesetzt werden.

In dem erfindungsgemäßen Verfahren werden vorzugsweise Ruthenium-Trägerkatalysatoren mit 0,5 bis 3 Massen-% Ru auf oberflächenreichen Aluminiumoxiden als Träger verwendet.

Die Katalysatoren werden zweckmäßig in einer Form eingesetzt, die einen geringen Strömungswiderstand bietet, z. B. in Form von Granalien, Pellets oder Formkörpern, wie Tabletten, Zylindern, Kugeln, Strangextudaten oder Ringen. Sie werden zweckmäßig vor ihrem Einsatz durch Erhitzen im Wasserstoffstrom, beispielsweise auf 140 bis 250 °C, aktiviert, sofern sie nicht im Hydrierreaktor reduziert werden. Beispielsweise wird ein Verfahren zur Reduktion eines Katalysators mit Wasserstoff in Gegenwart einer flüssigen Phase in DE19933348A1 beschrieben.

Der Volumenanteil des ersten Katalysators am Gesamtkatalysatorvolumen beträgt vorzugsweise 70 bis 98 %, insbesondere ist das Volumen des ersten Katalysators mindestens dreimal so groß wie das Volumen des zweiten Katalysators. Ganz besonders bevorzugt ist der Volumenanteil des ersten Katalysators am Gesamtkatalysatorvolumen 80 bis 95 %, ganz besonders bevorzugt 90 bis 95 %.

In einer ganz besonders bevorzugten Ausführungsform des erfindungsgemäßen Verfahrens wird in der ersten Hydrierstufe ein Katalysator aus lediglich Kupfer und Nickel auf einem Träger, d. h. frei von Chrom und/oder Bariumoxid, und in der zweiten Hydrierstufe ein Pd-haltiger Trägerkatalysator eingesetzt. Durch diese Katalysatorkombination werden besonders gute Hydrierergebnisse in dem erfindungsgemäßen Verfahren erzielt, d. h. der Anteil der Decenale kann besonders stark gesenkt werden, bei gleichzeitiger guter Recyclierbarkeit der Katalysatoren, was insgesamt zu einem besonders wirtschaftlichen Prozess führt. Im erfindungsgemäßen Verfahren wird die Hydrierung der Decenalgemische zu den entsprechenden gesättigten C₁₀-Alkoholen an zwei in unterschiedlichen Reaktoren oder Betten angeordneten Katalysatoren durchgeführt. Optional können beide Hydrierstufen nacheinander in einem Reaktor erfolgen. Dies bedeutet, dass die unterschiedlichen Katalysatoren in einem Reaktor in verschiedenen Betten angeordnet sind. In der einfachsten Ausführungsform wird das erfindungsgemäße Verfahren in einem zwei Betten aufweisenden Reaktor durchgeführt, wobei das eine Bett von dem ersten Katalysator gebildet wird und das andere Bett von dem zweiten Katalysator gebildet wird.

Im erfindungsgemäßen Verfahren werden Verfahrensausführungen mit mindestens zwei Reaktoren bevorzugt, wobei der zweite Reaktor ein oder zwei Betten aufweisen kann.

Vorzugsweise wird das erfindungsgemäße Verfahren in mindestens zwei hintereinander geschalteten Reaktoren durchgeführt, wobei der erste Reaktor den ersten Katalysator enthält und der zweite Reaktor den zweiten Katalysator enthält, und wobei die erste Hydrierung im ersten Reaktor erfolgt und die zweite Hydrierung im zweiten Reaktor erfolgt.

Für jede Reaktionszone des erfindungsgemäßen Verfahrens können unterschiedliche Verfahrensvarianten gewählt werden. Es kann adiabatisch, oder praktisch isotherm, d. h. mit einem Temperaturanstieg kleiner 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben.

Die beiden Reaktionszonen des erfindungsgemäßen Verfahrens werden bevorzugt kontinuierlich und bevorzugt in der Rieselphase oder in der Flüssigphase in Dreiphasenreaktoren im Gleichstrom durchgeführt, wobei der Wasserstoff in an sich bekannter Weise in dem flüssigen Aldehydstrom fein verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor isotherm und im geraden Durchgang betrieben, so kann die spezifische Katalysatorbelastung (LHSV = liquid hourly space velocity) Werte zwischen 0,1 und 10 h⁻¹ annehmen.

Eine spezielle Ausführung des erfindungsgemäßen Verfahrens ist dadurch gekennzeichnet, dass der erste Reaktor mit dem ersten Katalysator in Schlaufenfahrweise (externe Rückführung) und der zweite Reaktor, der entweder nur den zweiten Katalysator oder in der oberen Reaktionszone den ersten Katalysator und in der unteren Reaktionszone den zweiten Katalysator enthält, im geraden Durchgang betrieben wird.

Optional kann die Hydrierung auch in drei hintereinander geschalteten Reaktoren durchgeführt werden. Dabei wird der erste Reaktor mit einem Teil des ersten Katalysators vorzugsweise in Schlaufenfahrweise (externe Rückführung) betrieben, der zweite Reaktor mit dem Rest des ersten Katalysators und der dritte Reaktor mit dem zweiten Katalysator werden im geraden Durchgang betrieben. Hierbei erfolgt die erste Hydrierung im ersten und im zweiten Reaktor und die zweite Hydrierung erfolgt im dritten Reaktor.

Zur Minimierung von Nebenreaktionen und somit Erhöhung der Ausbeute ist es zweckmäßig, die Aldehydkonzentration im Reaktorzulauf zu begrenzen. Insbesondere liegt der Anteil an Decenal im Zulauf des ersten Reaktors zwischen 1 bis 35 Massen-%, bevorzugt zwischen 5 und 25 Massen-%. Der gewünschte Konzentrationsbereich kann bei Reaktoren, die in Schlaufenfahrweise betrieben werden, durch die Kreislaufrate (Mengenverhältnis von zurückgeführtem Hydrieraustrag zu Edukt) eingestellt werden.

Die zwei oder mehreren Stufen des erfindungsgemäßen Verfahrens werden in einem Druckbereich von 0,5 bis 10 MPa, insbesondere zwischen 0,5 und 4 MPa, ganz besonders im Bereich von 1 bis 2,5 MPa durchgeführt. Die Hydriertemperaturen liegen zwischen 120 und 220 °C, insbesondere zwischen 140 und 190 °C. Die einzelnen Stufen werden bei gleichem Druck oder unterschiedlichen Druck betrieben. Die Temperaturen sind in den einzelnen Stufen gleich oder verschieden.

Der für die Hydrierung eingesetzte Wasserstoff kann inerte Gase wie beispielsweise Methan oder Stickstoff enthalten. Vorzugsweise wird Wasserstoff mit einer Reinheit größer als 98 %, insbesondere größer als 99 % eingesetzt.

Jeder Reaktor kann mit Frisch-Wasserstoff betrieben werden. Es ist jedoch auch möglich, dass das Abgas eines Reaktors ganz oder teilweise der Einsatzwasserstoff eines anderen Reaktor sein kann. Im letzteren Fall kann flüssiges Hydriergut (Edukt/Produkte) und Wasserstoff in gleicher oder umgekehrter Reihenfolge durch die Reaktoren strömen.

Das Hydrierprodukt wird destillativ aufgearbeitet. Dies geschieht bei Normaldruck oder vorzugsweise bei vermindertem Druck. Vorzugsweise wird das Hydriergemisch in drei Fraktionen getrennt, nämlich Vorlauf, Decanolfraktion (gesättigte C₁₀-Alkohole) und Hochsiederfraktion (Stoffe, die höher als die Decanole sieden) getrennt. Enthält der Vorlauf noch C₁₀-Aldehyde, kann dieser teilweise in die Hydrierung zurückgeführt werden.

Die C₁₀-Alkohole werden, wie bereits erwähnt, vorzugsweise für die Herstellung von Weichmachern oder Detergenzien verwendet.

Die folgenden Beispiele sollen die Erfindung erläutern, ohne die Anwendungsbreite einzuschränken, die sich aus der Beschreibung und den Patentansprüchen ergibt.

### Beispiel 1 (nicht erfindungsgemäß):

### Hydrierung mit erstem Katalysator (Cu/Cr/Ni auf Al₂O₃-Träger)

Ein Reaktionsaustrag aus der kontinuierlichen Aldolisierung von n-Pentanal und 2-Methylbutanal mit rund 88 Massen-% 2-Propylheptenal und rund 3,2 Massen-% 4-Metyl-2-Propyl-Hexenal wurde in einer Kreislaufapparatur bei 180 °C und 25 bar absolut an 105,3 g (entsprechend 150 ml) eines Cu/Cr/Ni-Katalysators auf Al₂O₃-Träger (Strangextrudate:1,5 mm Durchmesser, 3-5 mm Länge) mit 6 Massen-% Kupfer, 3,1 Massen-% Nickel und 0,6 Massen-% Chrom in der Flüssigphase kontinuierlich hydriert. Es wurden stündlich 0,10 l Edukt, entsprechen einem LHSV-Wert von 0,66 h⁻¹ bei einem Kreislauf von 40 l/h durchgesetzt. Die Abgasmenge betrug 60 Nl/h. Die Edukt- und Produkt-Analysen in Massen % sind in Tabelle 1 wiedergegeben.

**Tabelle 1: Hydrierung von C₁₀-Aldehyden am Cu/Cr/Ni-Katalysator**

| **Zeit / h** | **0** | **500** | **1000** | **3000** |
|---|---|---|---|---|
| 2-Methylbutanal | 2,48 | 0,03 | 0,05 | 0,04 |
| n-Pentanal | 2,98 | 0,03 | 0,05 | 0,04 |
| 2-Methylbutanol | 0,01 | 2,92 | 2,76 | 2,96 |
| n-Pentanol | 0,09 | 3,70 | 3,44 | 3,71 |
| Zwischenlauf * | 0,01 | 1,03 | 1,19 | 1,21 |
| 4-Methyl-2-Propyl-Hexenal | 3,20 | 0,03 | 0,05 | 0,04 |
| 2-Propylheptanal | 0,00 | 0,25 | 0,44 | 0,69 |
| 4-Methyl-2-Propyl-Hexanal | 0,00 | 0,03 | 0,05 | 0,06 |
| 4-Methyl-2-Propyl-Hexanol | 0,00 | 3,14 | 3,15 | 3,11 |
| 4-Methyl-2-Propyl-Hexenol | 0,00 | 0,01 | 0,01 | 0,01 |
| 2-Propylheptenal | 87,99 | 0,35 | 0,47 | 0,85 |
| 2-Propylheptanol | 0,00 | 86,55 | 85,89 | 85,20 |
| 2-Propylheptenol | 0,01 | 0,01 | 0,03 | 0,09 |
| Hochsieder | 1,68 | 1,43 | 1,53 | 1,41 |
| Rest/GC | 1,56 | 0,73 | 0,89 | 0,58 |

| | | | | |
|---|---|---|---|---|
| *Zwischenlauf (unter anderen C₅-Ester und C₅-Säuren) | | | | |

Wie man aus der Tabelle 1 entnehmen kann, wurden die beiden ungesättigten C₁₀-Aldehyde 2-Propylheptenal und 4-Methyl-2-Propyl-Hexenal am Cu/Cr/Ni-Standardkatalysator mit hoher Selektivität zu den Wertprodukten 2-Propyheptanol und 4-Methyl-2-Propyl-Hexanol umgesetzt.

Die im Edukt vorhandenen, nicht umgesetzten C₅-Aldehyde (n-Pentanal und 2-Methylbutanal) wurden praktisch vollständig zu entsprechenden C₅-Alkoholen umgesetzt.

Die Restgehalte an ungesättigtem C₁₀-Aldehyd 2-Propylheptenal stiegen von 0,35 Gew.-% zu Beginn der Hydrierung nach 500 Stunden auf rund 0,85 Gew.-% nach 3000 Versuchstunden.

Im Produktspektrum der Hydrierung am Cu/Cr/Ni-Katalysator wurde das Zwischenprodukt der 2-Propylheptenal-Hydrierung, der gesättigte C₁₀-Aldehyd 2-Propylheptanal, nachgewiesen.

### Beispiel 2 (nicht erfindungsgemäß):

### Hydrierung mit erstem Katalysator (Cu/Ni auf Al₂O₃-Träger)

Ein Reaktionsaustrag aus der kontinuierlichen Aldolisierung von n-Pentanal und 2-Methylbutanal mit rd. 86,5 Massen-% 2-Propylheptenal und 3,3 Massen-% 4-Methyl-2-Propyl-Hexenal wurde in einer Kreislaufapparatur bei 180 °C und 25 bar absolut an 109,8 g (entsprechend 150 ml) eines Cu/Ni-Katalysators auf Al₂O₃-Träger (gleicher Träger wie im Beispiel 1) mit 9,5 Massen-% Kupfer und 6,1 Massen-% Nickel in der Flüssigphase kontinuierlich hydriert. Es wurden stündlich 0,10 l Edukt, entsprechen einem LHSV-Wert von 0,66 h⁻¹ bei einem Kreislauf von 40 l/h durchgesetzt. Die Abgasmenge betrug 60 Nl/h. Die Edukt- und Produkt-Analysen in Massen % sind in Tabelle 2 wiedergegeben.

**Tabelle 2: Hydrierung von C₁₀-Aldehyden am Cu/Ni-Katalysator**

| **Zeit / h** | **0** | **500** | **1000** | **3000** |
|---|---|---|---|---|
| 2-Methylbutanal | 2,89 | 0,03 | 0,03 | 0,02 |
| n-Pentanal | 4,03 | 0,04 | 0,04 | 0,04 |
| 2-Methylbutanol | 0,02 | 2,99 | 2,99 | 2,98 |
| n-Pentanol | 0,14 | 4,65 | 4,75 | 4,80 |
| Zwischenlauf * | 0,01 | 1,35 | 1,34 | 1,32 |
| 4-Methyl-2-Propyl-Hexenal | 3,30 | 0,01 | 0,00 | 0,01 |
| 2-Propylheptanal | 0,14 | 0,01 | 0,01 | 0,01 |
| 4-Methyl-2-Propyl-Hexanal | 0,00 | 0,09 | 0,06 | 0,05 |
| 4-Methyl-2-Propyl-Hexanol | 0,00 | 2,91 | 2,90 | 2,90 |
| 4-Methyl-2-Propyl-Hexenol | 0,00 | 0,01 | 0,01 | 0,01 |
| 2-Propylheptenal | 86,67 | 0,19 | 0,34 | 0,65 |
| 2-Propylheptanol | 0,05 | 85,54 | 85,46 | 85,10 |
| 2-Propylheptenol | 0,01 | 0,00 | 0,00 | 0,02 |
| Hochsieder | 1,72 | 1,43 | 1,44 | 1,54 |
| Rest /GC | 1,03 | 0,76 | 0,63 | 0,55 |

| | | | | |
|---|---|---|---|---|
| *Zwischenlauf (unter anderen C₅-Ester und C₅-Säuren) | | | | |

Unter den gewählten Reaktionsbedingungen stiegen die Restgehalte an ungesättigtem C₁₀-Aldehyd 2-Propylheptenal von 0,19 Gew.-% nach 500 Stunden auf rund 0,65 Gew.-% nach 3000 Versuchstunden. Dieses Ergebnis ist mit dem Hydrierergebnis in Beispiel 1 vergleichbar.

Im Unterschied zu Cu/Cr/Ni-Katalysator (Beispiel 1) wird am Cu/Ni-Katalysator mit deutlich höheren Cu- und Ni-Gehalten das Zwischenprodukt der 2-Propylheptenal-Hydrierung, der gesättigte C₁₀-Aldehyd 2-Propylheptanal, praktisch vollständig zum Wertprodukt 2-Propylheptanol umgesetzt.

### Beispiel 3 (erfindungsgemäß):

### Hydrierung mit einer Kombination aus Cu/Cr/Ni-Katalysator und Ru-Katalysator

Im folgenden Beispiel sind die Ergebnisse der Hydrierung gemäß der vorliegenden Erfindung dargestellt. Statt eines Katalysators bestand das Katalysatorbett aus einer Kombination zweier Katalysatoren. Das Katalysator-Gesamtvolumen betrug wie im Beispiel 1 150 ml.

Der Hauptanteil des Katalysatorbettes stellte mit 120 ml der Cu/Cr/Ni-Katalysator, welcher auch im Beispiel 1 verwendet wurde, dar. Dieser Katalysator wurde an der Anströmseite des Reaktors platziert.

Dem Cu/Cr/Ni-Katalysator (Strangextrudate:1,5 mm Durchmesser, 3-5 mm Länge) folgte ein Ruthenium-Trägerkatalysator (1,5 Massen-% Ru auf γ-Aluminiumoxid als Träger mit 30 ml Katalysatorvolumen.

Für die Hydrierung wurde Edukt gleicher Zusammensetzung wie im Beispiel 1 verwendet.

Die Reaktionsbedingungen der Hydrierung mit 180 °C, 25 bar und einem Eduktdurchsatz von 0,10 l/h bei einem Kreislauf von 40 l/h, waren mit Bedingungen der Hydrierung im Beispiel 1 vergleichbar.

Die Ergebnisse der Hydrierung an der erfindungsgemäßen

Katalysatorkombination sind in Tabelle 3 dargestellt.

**Tabelle 3: Hydrierung an Cu/Cr/Ni- und Ru-Katalysatorkombination**

| **Zeit / h** | **0** | **500** | **1000** | **3000** |
|---|---|---|---|---|
| 2-Methylbutanal | 2,48 | 0,03 | 0,05 | 0,05 |
| n-Pentanal | 2,98 | 0,03 | 0,05 | 0,04 |
| 2-Methylbutanol | 0,01 | 2,98 | 2,77 | 2,98 |
| n-Pentanol | 0,09 | 3,70 | 3,54 | 3,71 |
| Zwischenlauf * | 0,01 | 1,17 | 1,22 | 1,21 |
| 4-Methyl-2-Propyl-Hexenal | 3,20 | 0,03 | 0,07 | 0,01 |
| 2-Propylheptanal | 0,00 | 0,64 | 0,82 | 1,39 |
| 4-Methyl-2-Propyl-Hexanal | 0,00 | 0,08 | 0,08 | 0,05 |
| 4-Methyl-2-Propyl-Hexanol | 0,00 | 3,15 | 3,21 | 3,11 |
| 4-Methyl-2-Propyl-Hexenol | 0,00 | 0,01 | 0,01 | 0,01 |
| 2-Propylheptenal | 87,99 | 0,03 | 0,04 | 0,07 |
| 2-Propylheptanol | 0,00 | 86,56 | 85,93 | 85,21 |
| 2-Propylheptenol | 0,01 | 0,01 | 0,03 | 0,09 |
| Hochsieder | 1,68 | 1,49 | 1,42 | 1,41 |
| Rest /GC | 1,56 | 0,72 | 0,76 | 0,58 |

| | | | | |
|---|---|---|---|---|
| *Zwischenlauf (unter anderen C₅-Ester und C₅-Säuren) | | | | |

Die Ergebnisse in Tabelle 3 zeigen, dass die Ausbeuten der 2-Propylheptenal-Hydrierung in Gegenwart der erfindungsgemäßen Katalysatorkombination mit den am Standardkatalysator erzielbaren Ausbeuten im Beispiel 1 vergleichbar sind. Nach der vorliegenden Analyse der Reaktionsausträge liegen die Gehalte an gewünschten Wertprodukt 2-Propylheptanol (2-PH-ol) in beiden Fällen zwischen 85 und 86 Massen-%.

Im Unterschied zur Standardfahrweise mit einem Katalysator im Beispiel 1 mit steigenden Gehalten an ungesättigtem C₁₀-Alehyd 2-Propylheptenal, wurden die 2-Propylheptenal-Gehalte im Reaktionsaustrag bei dem Einsatz der Katalysatorkombination deutlich reduziert.

Nach einer Versuchszeit von rund 3000 Stunden wurde ein geringer Gehalt an 2-Propylheptenal von rund 0,07 Massen-%, entsprechend 700 ppm, ermittelt. Nach den vorliegenden Ergebnissen wurde das 2-Propylheptenal vorzugsweise zu 2-Propylheptanal umgesetzt.

### Beispiel 4 (nicht erfindungsgemäß):

### Hydrierung mit einer Kombination aus Cu/Ni-Katalysator und Pd-Katalysator

Im folgenden Beispiel sind die Ergebnisse der Hydrierung gemäß der vorliegenden Erfindung unter Verwendung einer zweiten Katalysatorkombination dargestellt, die aus einem Cu/Ni-Katalysator als erster Katalysator und einem nachgeschalteten Palladium-Katalysator bestand. Wie im Beispiel 2 betrug das Katalysator-Gesamtvolumen 150 ml.

Unter Einhaltung des gleichen Volumenverhältnisses wie im Beispiel 3 wurde der Reaktor von der Anströmseite her mit 120 ml des Cu/Ni-Katalysators und mit 30 ml des Palladium-Katalysators (0,5 Massen-% Pd) gleicher geometrischer Form (Extrudate: 1,5 mm Durchmesser, 2 bis 4 mm Länge) befüllt.

Für die Hydrierung wurde Edukt gleicher Zusammensetzung wie in Beispiel 2 verwendet. Die Hydrierung erfolgte unter gleichen Reaktionsbedingungen wie in den vorangegangenen Beispielen, d. h. bei 180 °C, 25 bar und 100 ml/h C₉-Aldehyd-Durchsatz.

Die Ergebnisse der Hydrierung an der zweiten erfindungsgemäßen Katalysatorkombination sind in Tabelle 4 dargestellt.

**Tabelle 4: Hydrierung an Cu/Ni- und Pd-Katalysatorkombination**

| **Zeit / h** | **0** | **500** | **1000** | **3000** |
|---|---|---|---|---|
| 2-Methylbutanal | 2,89 | 0,03 | 0,03 | 0,02 |
| n-Pentanal | 4,03 | 0,04 | 0,04 | 0,04 |
| 2-Methylbutanol | 0,02 | 2,99 | 2,99 | 2,98 |
| n-Pentanol | 0,14 | 4,68 | 4,79 | 4,86 |
| Zwischenlauf * | 0,01 | 1,35 | 1,34 | 1,32 |
| 4-Methyl-2-Propyl-Hexenal | 3,30 | 0,01 | 0,00 | 0,01 |
| 2-Propylheptanal | 0,14 | 0,18 | 0,22 | 0,41 |
| 4-Methyl-2-Propyl-Hexanal | 0,00 | 0,08 | 0,07 | 0,05 |
| 4-Methyl-2-Propyl-Hexanol | 0,00 | 2,91 | 2,90 | 2,91 |
| 4-Methyl-2-Propyl-Hexenol | 0,00 | 0,01 | 0,01 | 0,01 |
| 2-Propylheptenal | 86,67 | 0,01 | 0,02 | 0,04 |
| 2-Propylheptanol | 0,05 | 85,54 | 85,48 | 85,25 |
| 2-Propylheptenol | 0,01 | 0,00 | 0,00 | 0,00 |
| Hochsieder | 1,72 | 1,43 | 1,47 | 1,52 |
| Rest /GC | 1,03 | 0,74 | 0,63 | 0,58 |

Wie man aus der Tabelle 4 entnehmen kann, wurde bei der Hydrierung von ungesättigten C₁₀-Aldehyden in Gegenwart von Cu/Ni- und Pd-Katalysatorkombination vergleichbare Ausbeuten wie bei dem Einsatz des Cu/Ni-Katalysatorsystems in Beispiel 2 erzielt.

Im Vergleich zu Beispiel 2 mit einem Katalysator wurden die Restgehalte an C₁₀-Aldehyd 2-Propylheptenal im Produktaustrag durch den nachgeschalteten Pd-Katalysator deutlich reduziert. Nach rund 3000 Versuchstunden wurde ein 2-Propylheptenal-Gehalt von rund 400 ppm ermittelt.

## Patentansprüche

1. Verfahren zur Herstellung mindestens eines Decanols durch Hydrierung mindestens eines α,β-ungesättigten Decenals, bei welchem eine erste Hydrierung in der flüssigen Phase an einem festen ersten Katalysator erfolgt, wobei der erste Katalysator Kupfer und Nickel enthält,
**gekennzeichnet durch**
eine auf die erste Hydrierung folgende zweite Hydrierung in der flüssigen Phase an einem festen zweiten Katalysator, wobei der zweite Katalysator frei von Kupfer, Chrom und Nickel ist und Ruthenium enthält.

2. Verfahren nach Anspruch 1,
**dadurch gekennzeichnet,**
**dass** der Volumenanteil des ersten Katalysators am Gesamtkatalysatorvolumen 70 bis 98 % beträgt.

3. Verfahren nach Anspruch 2,
**dadurch gekennzeichnet,**
**dass** das Volumen des ersten Katalysators mindestens dreimal so groß ist wie das Volumen des zweiten Katalysators.

4. Verfahren nach einem der Ansprüche 1 bis 3,
**dadurch gekennzeichnet,**
**dass** es in mindestens zwei hintereinander geschalteten Reaktoren durchgeführt wird, wobei der erste Reaktor den ersten Katalysator enthält und der zweite Reaktor den zweiten Katalysator enthält, und wobei die erste Hydrierung im ersten Reaktor erfolgt und die zweite Hydrierung im zweiten Reaktor erfolgt.

5. Verfahren nach Anspruch 4,
**dadurch gekennzeichnet,**
**dass** es in drei hintereinander geschalteten Reaktoren durchgeführt wird, wobei der erste Reaktor einen Teil des ersten Katalysators enthält und in Schlaufenfahrweise betrieben wird, wobei der zweite Reaktor den Rest des ersten Katalysators enthält, wobei der dritte Reaktor den zweiten Katalysator enthält, wobei die erste Hydrierung im ersten Reaktor und im zweiten Reaktor erfolgt und wobei die zweite Hydrierung im dritten Reaktor erfolgt.

6. Verfahren nach einem der Ansprüche 1 bis 3,
**dadurch gekennzeichnet,**
**dass** es in einem zwei Betten aufweisenden Reaktor durchgeführt wird, wobei das eine Bett von dem ersten Katalysator gebildet wird und das andere Bett von dem zweiten Katalysator gebildet wird.

7. Verfahren nach einem der Ansprüche 1 bis 6,
**dadurch gekennzeichnet,**
**dass** der erste Katalysator frei von Bariumoxid ist.

8. Verfahren nach einem der Ansprüche 1 bis 7,
**dadurch gekennzeichnet,**
**dass** der erste Katalysator frei von Chrom ist.

## Claims

1. Process for preparing at least one decanol by hydrogenation of at least one α,β-unsaturated decenal, in which a first hydrogenation is effected in the liquid phase over a solid first catalyst, said first catalyst comprising copper and nickel,
**characterized by** a second hydrogenation, following the first hydrogenation, in the liquid phase over a solid second catalyst, said second catalyst being free of copper, chromium and nickel and comprising ruthenium.

2. Process according to Claim 1,
**characterized in that**
the proportion by volume of the first catalyst in the total catalyst volume is 70 to 98%.

3. Process according to Claim 2,
**characterized in that**
the volume of the first catalyst is at least three times as large as the volume of the second catalyst.

4. Process according to any of Claims 1 to 3,
**characterized in that**
it is performed in at least two reactors connected in series, the first reactor containing the first catalyst and the second reactor containing the second catalyst, and the first hydrogenation being effected in the first reactor and the second hydrogenation being effected in the second reactor.

5. Process according to Claim 4,
**characterized in that**
it is performed in three reactors connected in series, the first reactor containing a portion of the first catalyst and being operated in loop mode, the second reactor containing the rest of the first catalyst, the third reactor containing the second catalyst, the first hydrogenation being effected in the first reactor and in the second reactor, and the second hydrogenation being effected in the third reactor.

6. Process according to any of Claims 1 to 3,
**characterized in that**
it is performed in a reactor having two beds, one bed being formed by the first catalyst and the other bed being formed by the second catalyst.

7. Process according to any of Claims 1 to 6,
**characterized in that**
the first catalyst is free of barium oxide.

8. Process according to any of Claims 1 to 7,
**characterized in that**
the first catalyst is free of chromium.

## Revendications

1. Procédé pour la préparation d'au moins un décanol par hydrogénation d'au moins un décénal α,β-insaturé, dans lequel une première hydrogénation a lieu en phase liquide sur un premier catalyseur solide, le premier catalyseur contenant du cuivre et du nickel, **caractérisé par** une deuxième hydrogénation, suivant la première hydrogénation, en phase liquide sur un deuxième catalyseur solide, le deuxième catalyseur étant exempt de cuivre, de chrome et de nickel et contenant du ruthénium.

2. Procédé selon la revendication 1, **caractérisé en ce que** la proportion en volume du premier catalyseur par rapport au volume total de catalyseur est de 70 à 98%.

3. Procédé selon la revendication 2, **caractérisé en ce que** le volume du premier catalyseur est au moins trois fois plus grand que le volume du deuxième catalyseur.

4. Procédé selon l'une quelconque des revendications 1 à 3, **caractérisé en ce qu'**il est réalisé dans au moins deux réacteurs disposés l'un derrière l'autre, le premier réacteur contenant le premier catalyseur et le deuxième réacteur contenant le deuxième catalyseur et la première hydrogénation ayant lieu dans le premier réacteur et la deuxième hydrogénation ayant lieu dans le deuxième réacteur.

5. Procédé selon la revendication 4, **caractérisé en ce qu'**il est réalisé dans trois réacteurs disposés les uns derrière les autres, le premier réacteur contenant une partie du premier catalyseur et étant exploité dans un mode opératoire en boucle, le deuxième réacteur contenant le reste du premier catalyseur, le troisième réacteur contenant le deuxième catalyseur, la première hydrogénation ayant lieu dans le premier réacteur et dans le deuxième réacteur et la deuxième hydrogénation ayant lieu dans le troisième réacteur.

6. Procédé selon l'une quelconque des revendications 1 à 3, **caractérisé en ce qu'**il est réalisé dans un réacteur présentant deux lits, un lit étant formé par le premier catalyseur et l'autre lit par le deuxième catalyseur.

7. Procédé selon l'une quelconque des revendications 1 à 6, **caractérisé en ce que** le premier catalyseur est exempt d'oxyde de baryum.

8. Procédé selon l'une quelconque des revendications 1 à 7, **caractérisé en ce que** le premier catalyseur est exempt de chrome.
